# EUROPEAN PATENT APPLICATION

(11) **EP 3 904 373 A1**
(43) Date of publication of application: **03.11.2021**
(21) Application number: 19906367.8
(22) Date of filing: 26.12.2019
(51) Int. Cl.: C07K 14/62, C12P 21/00

(54) **COMPOSITION FOR CONVERTING INSULIN PRECURSOR INTO INSULIN ENZYME AND METHOD FOR CONVERTING INSULIN PRECURSOR INTO INSULIN BY USING SAME**

(30) Priority: 27.12.2018 KR 20180170529
(71) Applicant: Polus Inc., Incheon 21984 (KR)
(72) Inventor: KIM, Jinhwan, Incheon 21988 (KR); BYUN, Seungmin, Bucheon-si Gyeonggi-do 14597 (KR); JO, Munjeong, Incheon 21561 (KR); KIM, Hyunuk, Incheon 21986 (KR); KIM, Dongkyu, Incheon 21986 (KR)
(74) Representative: Budde Schou A/S
(86) International application number: PCT/KR2019/018465
(87) International publication number: WO 2020/138952

(57) **Abstract**

The present invention relates to: a composition for an enzymatic conversion reaction for converting proinsulin or a proinsulin analogue into insulin or an insulin analogue, the composition comprises one or more selected from the group consisting of calcium chloride, glycine, and proline, and trypsin and/or carboxypeptidase B; and a method for converting proinsulin or a proinsulin analogue into insulin or an insulin analogue by using the composition. The present invention increases the structural stability of an insulin protein and the stability of the enzyme at the same time so as to significantly improve the enzymatic conversion yield and the purity of insulin, thereby being useful in insulin production processes.

## Description

### [Technical Field]

The present invention relates to a composition for enzymatic conversion of an insulin precursor into insulin and a method for converting an insulin precursor into insulin using the same, and more particularly to a composition for enzymatic conversion into insulin, which is added along with an enzyme in order to increase the yield and purity of insulin in an enzymatic reaction for converting an insulin precursor into insulin, and a method for converting an insulin precursor into insulin using the same.

### [Background Art]

Diabetes is a metabolic disease characterized by high blood sugar, and is caused by the complex action of genetic and environmental factors. Diabetes causes conditions such as type 1 diabetes, type 2 diabetes, gestational diabetes, hyperglycemia and the like, and is a metabolic disorder in which the pancreas produces an insufficient amount of insulin or in which cells of the human body do not respond properly to insulin, resulting in decreased ability to uptake glucose, and consequently, glucose accumulates in the blood.

The most representative method for the treatment of diabetes is a method of controlling a patient's blood sugar to a normal level by administering insulin. Insulin is a blood sugar control hormone secreted by the pancreas of the human body, and it plays a role in moving excess glucose in the blood to the cells so as to supply the cells with an energy source while maintaining blood sugar at a normal level.

With the development of technology for manufacturing a recombinant protein, various types of insulin products have been launched, and depending on the reactivity thereof, insulin products may be broadly classified into five types, namely as rapid-acting insulin, short-acting insulin, intermediate-acting insulin, mixed insulin, and long-acting insulin. Among these, rapid-acting insulin, which shows the fastest response, begins to act between 1 minute and 20 minutes due to the fast effect thereof, and exhibits the best effect after about 1 hour, and the effect thereof lasts for 3 to 5 hours, and representative examples thereof include insulin aspart (NovoRapid^{®}), insulin lispro (Humalog^{®}), and insulin glulisine (Apidra^{®}). The next-fastest-acting insulin is short-acting insulin, and short-acting insulin begins to lower blood sugar level about 30 minutes after administration and shows the best effect between 2 and 4 hours, and the effect thereof lasts for 6 to 8 hours. Representative examples of short-acting insulin include Actrapid^{®}, Humilin^{®}, Hypurin Neutral, and the like. Intermediate-acting insulin, containing protamine or zinc to prolong the action of insulin, begins to act about 1 hour and 30 minutes after injection, and the effect thereof reaches the maximum level between 4 and 12 hours and lasts for 16 to 24 hours. Representative examples thereof include Protaphane^{®}, Humulin^{®} NPH, and Hypurin Isophane^{®}. Mixed insulin is a pre-mixed combination of rapid-acting insulin or short-acting insulin with intermediate-acting insulin so that two types of insulin may be easily administered through a single injection, and NovoMix^{®} 30 (30% insulin aspart, 70% protamine crystallized insulin aspart), Humalog^{®} Mix 25 (25% insulin lispro, 75% insulin lispro protamine suspension), and Humalog^{®} Mix 50 (50% insulin lispro, 50% insulin lispro protamine suspension) are commercially available. Long-acting insulin is an insulin, which is injected once or twice a day and in which the effect thereof lasts up to 24 hours, and is usually used as a basal insulin, and Lantus^{®} (insulin glargine, EP 0368187), Levemir^{®} (insulin detemir, US 5,750,497), and Tresiba^{®} (insulin degludec, US 7,615,532) are marketed.

Meanwhile, insulin is subjected to various post-translational modifications depending on the production pathway thereof. Production and secretion thereof are independent, and produced insulin is stored for secretion. C-peptide and mature insulin exhibit biological activity.

In mammals, insulin is synthesized in the beta cells of the pancreas, and insulin is composed of two polypeptide chains, namely an A-chain and a B-chain, which are linked by disulfide bonds. Early insulin is synthesized into a single polypeptide called preproinsulin in beta cells. Preproinsulin contains a signal peptide of 24 amino acid residues that moves new polypeptide chains into the rough endoplasmic reticulum. The signal peptide induces movement into the lumen of the rough endoplasmic reticulum, followed by cleavage to form proinsulin. In the rough endoplasmic reticulum, proinsulin folds into the correct shape and forms three disulfide bonds. 5-10 minutes after assembly in the endoplasmic reticulum, proinsulin is transported into the trans-Golgi network, where immature granules are formed.

Proinsulin matures into active insulin by the activity of exoprotease carboxypeptidase E and cellular endopeptidases known as prohormone convertases (PC1, PC2). Endopeptidase induces cleavage at two positions to release a fragment called C-peptide, and two peptide chains, namely a B-chain and an A-chain, are linked by two disulfide bonds. Each cleavage site is located after a pair of basic residues (lysine (Lys)-64 and arginine (Arg)-65, and arginine (Arg)-31 and arginine (Arg)-32). After the C-peptide is cleaved, these two pairs of basic residues are removed by carboxypeptidase. C-peptide is located in the central portion of proinsulin, and the primary structure of proinsulin corresponds to "B-C-A" in that order (the B-chain and the A-chain were identified based on mass, and the C-peptide was later discovered).

The produced mature insulin (active insulin) is packaged in mature granules, and is secreted from the cells into the circulatory system by metabolic signals (e.g., leucine (Leu), arginine (Arg), glucose, mannose) and vagus nerve stimulation.

In order to treat diabetes, active insulin is administered. With regard to a gene-recombinant insulin production technology for producing active insulin, Eli Lilly and Company used a method in which the A-chain and the B-chain are expressed using *E. coli* and mixed *in vitro* to form a disulfide bond and the A- and B-chains are linked, but there is a problem in that the production efficiency is not good. Eli Lilly and Company subsequently devised a method of producing insulin by expressing proinsulin, forming a disulfide bond *in vitro,* and cleaving C-peptide with trypsin and carboxypeptidase B.

Novo Nordisk Inc. developed a method of obtaining insulin by expressing, in yeast, mini-proinsulin in which B- and A-chains are linked by two basic amino acids, followed by trypsinization under laboratory conditions. This method has the advantage of formation of a disulfide bond during expression and secretion of mini-proinsulin and of easy separation and purification due to secretion in the medium, but it is difficult to produce on as large a scale as when using *E. coli.*

The development of novel gene-recombinant insulin production methods has been thoroughly carried out since then. Hoechst AG developed a method of obtaining insulin in which a novel insulin derivative or preproinsulin is expressed in *E. coli* and a disulfide bond is formed under *in-vitro* conditions, followed by treatment with lysyl endopeptidase or clostripain and carboxypeptidase B, and Bio-Technology General Corporation improved the expression effect and the disulfide bond formation efficiency under *in-vitro* conditions by expressing a fusion protein in which proinsulin is linked to superoxide dismutase (SOD) in *E. coli.* Conversion into insulin was performed with trypsin and carboxypeptidase B. As described above, many attempts have been made to realize gene-recombinant insulin production methods, and improvements have been made in view of the expression efficiency, disulfide bond formation efficiency, and method of conversion into insulin (KR 10-2001-7013921).

The present inventors have made great efforts to develop a reaction composition suitable for enzymatic conversion of an insulin glargine precursor, having improved persistence due to the increased *in-vivo* half-life thereof compared to natural insulin, into insulin glargine, and as a result, have devised a composition for an enzymatic conversion reaction that is able to improve the structural stability of insulin and insulin analogues and the stability of the enzyme and ascertained that, when an enzymatic conversion reaction is carried out using the composition for an enzymatic conversion reaction, the yield and purity of insulin glargine are increased, thereby culminating in the present invention.

### [Disclosure]

It is an object of the present invention to provide a composition for enzymatic conversion suitable for an enzymatic process for converting an insulin precursor into insulin and an enzymatic conversion method using the composition.
In order to achieve the above and other objects, the present invention provides A composition for enzymatic converting an insulin precursor into insulin, comprising one or more selected from the group consisting of calcium chloride, glycine, and proline; and trypsin and/or carboxypeptidase B.

In addition, the present invention provides a method for converting an insulin precursor into insulin comprising adding an insulin precursor to the composition described above and carrying out a reaction.

### [Mode for Invention]

Unless otherwise defined, all technical and scientific terms used herein have the same meanings as those typically understood by those skilled in the art to which the present invention belongs. Generally, the nomenclature used herein and the test method described below are well known in the art and are typical.

In the present invention, when calcium chloride and an amino acid (proline or glycine) are added alone or in combination to a composition for an enzymatic conversion reaction converting an insulin glargine precursor into insulin glargine, it is confirmed that the yield of enzymatic conversion is increased and the purity of insulin is also increased.

Accordingly, an aspect of the present invention pertains to A composition for enzymatic converting an insulin precursor into insulin, comprising one or more selected from the group consisting of calcium chloride, glycine, and proline; and trypsin and/or carboxypeptidase B.

As used herein, the term "insulin precursor" refers to a single-stranded peptide including an insulin A-chain and an insulin B-chain, with a C-peptide therebetween, and may be used interchangeably with "proinsulin". In the present invention, the insulin precursor conceptually comprises all precursor forms such as natural insulin precursors, insulin analogue precursors, and derivatives thereof. The insulin precursor may be prepared by those of ordinary skill in the art with reference to methods disclosed in documents such as EP 0,211,299, EP 0,227,938, EP 0,229,998, EP 0,286,956, or KR 10-0158197.

As used herein, the term "insulin" refers to a protein that controls blood sugar in the body. Natural insulin is a hormone secreted by the pancreas, and typically promotes intracellular glucose uptake and inhibits the breakdown of fat, and thus plays a role in controlling blood sugar in the body. In the present invention, insulin conceptually comprises all forms such as natural insulin, insulin analogues, and derivatives thereof.

For insulin, an insulin precursor (proinsulin) having no blood sugar control function is processed into insulin having a blood sugar control function. Insulin is composed of two polypeptide chains, particularly an A-chain and a B-chain, each comprising 21 and 30 amino acid residues, which are linked by two disulfide bridges. The A-chain and B-chain of natural insulin may comprise the following amino acid sequences, respectively.

A-chain: 
Gly-Ile-Val-Glu-Gln-Cys-Cys-Thr-Ser-Ile-Cys-Ser-Leu-Tyr-

Gln-Leu-Glu-Asn-Tyr-Cys-Asn (SEQ ID NO: 1)
 B-chain:

Phe-Val-Asn-Gln-His-Leu-Cys-Gly-Ser-His-Leu-Val-Glu-Ala-
Leu-Tyr-Leu-Val-Cys-Gly-Glu-Arg-Gly-Phe-Phe-Tyr-Thr-Pro-
Lys-Thr (SEQ ID NO: 2)

The insulin precursor and insulin used in the present invention may be derived from human, but the present invention is not limited thereto.

In the present invention, the insulin analogue comprises one in which the amino acid of the B-chain or the A-chain is mutated compared to the native type. The *in-vivo* blood sugar control function of the insulin analogue may be the same as or may correspond to that of natural insulin. Specifically, the insulin analogue precursor or insulin analogue may be configured such that at least one amino acid of natural insulin is subjected to any variation selected from the group consisting of substitution, addition, deletion, modification, and combinations thereof, but the present invention is not limited thereto.

The insulin analogue that may be used in the present invention comprises an insulin analogue made by genetic recombination technology, and the insulin analogue conceptually comprises inverted insulin, insulin variants, insulin fragments, and the like.

The derivative has a blood sugar control function in the body, exhibits homology to each of the amino acid sequences of the A-chain and B-chain of the natural insulin or insulin analogue, and comprises a peptide in a form in which some groups of one amino acid residue are chemically substituted (e.g. alpha-methylation, alpha-hydroxylation), removed (e.g. deamination), or modified (e.g. N-methylation). The insulin fragment is a form in which at least one amino acid is added to or deleted from insulin, and the added amino acid may be an amino acid that does not exist in nature (e.g. a D-type amino acid), and such an insulin fragment plays a blood sugar control function in the body.

The insulin variant is a peptide having a sequence in which at least one amino acid is different from that of insulin, and plays a blood sugar control function in the body.

The insulin analogue, derivative, fragment and variant of the present invention may be used independently or in combination. For example, a peptide, which has a sequence in which at least one amino acid is different, in which the amino-terminal amino acid residue is subjected to deamination, and which plays a blood sugar control function in the body, is also comprised in the scope of the present invention.

Specifically, in the present invention, the insulin analogue may be insulin glargine. Insulin glargine is stabilized by substituting asparagine, which is the 21^{st} amino acid of the A-chain of insulin, with glycine, and is also made soluble at a weakly acidic pH by adding two arginines to the carboxy terminus of the B-chain. Here, insulin glargine is an insulin analogue developed such that it forms a microprecipitate in subcutaneous tissue when administered with an acidic solution (pH 4.0) and is slowly dissolved and released from the microprecipitate, which is an insulin glargine hexamer, whereby the action time is prolonged up to 24 hours. The A-chain and B-chain of insulin glargine may comprise the following amino acid sequences (US 5,656,722).

A-chain: 
Gly-Ile-Val-Glu-Gln-Cys-Cys-Thr-Ser-Ile-Cys-Ser-Leu-Tyr-

Gln-Leu-Glu-Asn-Tyr-Cys-Gly (SEQ ID NO:3)
 B-chain:

Phe-Val-Asn-Gln-His-Leu-Cys-Gly-Ser-His-Leu-Val-Glu-Ala-
Leu-Tyr-Leu-Val-Cys-Gly-Glu-Arg-Gly-Phe-Phe-Tyr-Thr-Pro-
Lys-Thr-Arg-Arg (SEQ ID NO: 4)

In the present invention, the term "enzymatic conversion" means converting an insulin precursor into insulin by digesting the insulin precursor with an enzyme. Specifically, enzymatic conversion serves to convert an insulin precursor (proinsulin) comprising a C-peptide between the A-chain and the B-chain into insulin using an enzyme, and the enzyme used for the enzymatic conversion may be selected from among trypsin, carboxypeptidase B, and combinations thereof. The term "enzymatic conversion" may be used interchangeably with terms such as "enzyme cleavage" or "enzyme digestion".

In the present invention, the composition may further comprise a buffer solution. The buffer solution for the enzymatic reaction may be 1 mM to 100 mM Tris-HCl or 1 mM to 100 mM borate, and preferably about 50 mM Tris-HCl or borate, but is not limited thereto.

The enzyme that is used in the present invention is preferably selected from among trypsin, carboxypeptidase B, and combinations thereof, but is not limited thereto.

In the present invention, the concentration of the insulin precursor that is allowed to react with the composition is 1 to 15 mg/mL, but is not limited thereto.

In the present invention, trypsin may be used at a weight ratio (w/w) of 1/1,000 to 1/40,000, preferably 1/1000 to 1/30,000 (w/w), more preferably 1/1,000 to 1/20,000 (w/w), and still more preferably 1/5,000 to 1/10,000 (w/w) relative to the insulin precursor, but the present invention is not limited thereto.

In the present invention, carboxypeptidase B may be used at a ratio of 1/600 to 1/20,000 (w/w), and preferably 1/600 to 1/15,000 (w/w) relative to the insulin precursor, but the present invention is not limited thereto.

Calcium chloride, glycine, and proline may be added each alone or in combination to the buffer solution of the enzymatic reaction of the present invention.

In the present invention, calcium chloride may be added at a concentration of 1 to 50 mM, preferably 5 to 40 mM, more preferably 10 to 40 mM, still more preferably 15 to 40 mM, even more preferably 20 to 35 mM, and most preferably 25 to 35 mM, but the present invention is not limited thereto.

In the present invention, glycine may be added at a concentration of 3 to 60 mM, preferably 10 to 60 mM, more preferably 20 to 60 mM, still more preferably 30 to 60 mM, and most preferably 40 to 60 mM, but the present invention is not limited thereto.

In the present invention, proline may be added at a concentration of 20 to 80 mM, preferably 20 to 70 mM, more preferably 20 to 60 mM, still more preferably 20 to 50 mM, and most preferably 20 to 40 mM, but the present invention is not limited thereto.

In another embodiment of the present invention, the composition may comprise 5-30 mM calcium chloride, 3-60 mM glycine, and 20-80 mM proline, each alone or in combination, but is not limited thereto.

Another aspect of the present invention pertains to a method for converting an insulin precursor into insulin comprising adding an insulin precursor to the composition and carrying out a reaction.

When the enzymatic conversion of the present invention, the pH of the reaction solution is not particularly limited, so long as effective conversion of the insulin precursor into insulin is possible, and the pH may be set to 7.5 to 9.5, and preferably 8.5 to 9.0, but is not limited thereto.

The reaction temperature in the enzymatic conversion of the present invention may be 4.0 to 25.0°C, but is not limited thereto.

The reaction time in the enzymatic conversion of the present invention may be 0.5 to 24 hours, but is not limited thereto.

A better understanding of the present invention may be obtained through the following examples. These examples are merely set forth to illustrate the present invention, and are not to be construed as limiting the scope of the present invention.

### Example 1. Preparation of insulin precursor

### 1-1. Refolding of insulin glargine precursor

In order to solubilize the insulin glargine precursor manufactured by the present applicant, 3.75 g of the insulin glargine precursor was added with a 0.2 M Tris-HCl buffer solution so that the volume thereof was adjusted to 250 mL, followed by stirring at room temperature for 1 hour. The solubilized solution was diluted 10-fold with a 0.2 M Tris-HCl buffer solution containing 0.4 mM cysteine as an oxidizing agent for refolding, and was then stirred at a low temperature for 10 hours. After completion of stirring, the pH of the resulting solution was lowered to 9.0 using hydrochloric acid.

### 1-2. Anion exchange chromatography purification

The solution containing the insulin glargine precursor prepared in Example 1-2 was bound to a POROS 50D (Thermo Scientific) ion resin equilibrated with a 50 mM Tris-HCl (pH 9.0) buffer solution, after which the insulin glargine precursor was eluted with a linear concentration gradient such that the concentration thereof was 10-14% using a 50 mM Tris-HCl (pH 9.0) buffer solution containing 0.5 M sodium chloride.

### 1-3. Citraconylation

This process is an optional process for reducing the amount of impurities that may occur during enzymatic conversion of the insulin glargine precursor, and was performed before enzyme treatment using the insulin glargine precursor purified in Example 1-2. The purified glargine precursor was diluted to a concentration of 1-15 mg/mL using a 50 mM Tris-HCl solution. Thereafter, citraconic anhydride was added to the diluted insulin glargine precursor solution such that the molar concentration ratio of citraconic anhydride to insulin glargine precursor was 1:9.9-266.5, and the pH was titrated to 8.5-9.0 using an aqueous sodium hydroxide solution, followed by stirring for 2 hours.

### Comparative Example 1. Enzymatic conversion without addition of salt or amino acid

The solution obtained in Example 1-3 was subjected to enzyme treatment to conduct an experiment for converting the insulin glargine precursor into insulin glargine. The insulin glargine precursor in the concentration range of 1 to 15 mg/mL was added with trypsin corresponding to 1/100,000-1/1,000 of the weight ratio of the protein, followed by stirring for 2 hours. After completion of stirring, the pH was titrated to 2.5 using acetic acid to complete the reaction. After completion of the reaction, the purity and yield of insulin glargine were analyzed using RP-HPLC.

### Example 2. Improvement in yield of enzymatic conversion reaction by addition of calcium chloride

In order to confirm the effect of the addition of calcium chloride on the yield of the enzymatic conversion reaction, the insulin glargine precursor obtained in Example 1-3 in the concentration range of 1 to 15 mg/mL was added with trypsin corresponding to 1/40,000-1/5,000 of the weight ratio of the protein, followed by stirring for 2 hours. Here, the reaction solution was 50 mM Tris-HCl, calcium chloride was added thereto in the concentration range of 1 to 20 mM, and the pH was titrated to 8.5-9.0. After completion of stirring, the pH was titrated to 2.5 using acetic acid to complete the reaction.

The purity and yield of the completed insulin glargine were analyzed using RP-HPLC as in Comparative Example 1. When calcium chloride was added during the enzymatic conversion reaction, the yield of the enzymatic conversion reaction was increased than when not added, and this effect was observed to be the highest when calcium chloride was added at a concentration of 20 mM (Table 1).

Table 1 below shows the yield of the enzymatic conversion reaction depending on the concentration of calcium chloride during the enzymatic conversion reaction of the insulin glargine precursor.

**[Table 1]**

| | Concentration (mM) | Purity (%) | Yield (%) |
|---|---|---|---|
| Comparative Example 1 | - | 33.4 | 39.5 |
| Calcium chloride | 1 | 35.5 | 41.6 |
| | 5 | 36.4 | 43.0 |
| | 10 | 37.0 | 43.3 |
| | 15 | 37.2 | 43.7 |
| | 20 | 37.5 | 44.1 |

As shown in Table 1, both the yield and the purity were increased depending on the concentration of calcium chloride. The purity was increased from 33.4% when calcium chloride was not added to 35.5-37.5% depending on the concentration thereof, and the yield was increased from 39.5% when calcium chloride was not added to 41.6-44.1% depending on the concentration thereof. From these results, it was found that the yield and purity were effectively increased when calcium chloride was added during the enzymatic conversion reaction.

### Example 3. Improvement in yield of enzymatic conversion reaction by addition of glycine

In order to confirm the effect of the addition of glycine on the yield of the enzymatic conversion reaction, the insulin glargine precursor obtained in Example 1-3 in the concentration range of 1 to 15 mg/mL was added with trypsin corresponding to 1/100,000-1/1,000 of the weight ratio of the protein, followed by stirring for 2 hours. Here, the reaction solution was 50 mM Tris-HCl, glycine was added thereto in the concentration range of 20 to 140 mM, and the pH was titrated to 8.5-9.0. After completion of stirring, the pH was titrated to 2.5 using acetic acid to complete the reaction.

The purity and yield of the completed insulin glargine were analyzed using RP-HPLC as in Comparative Example 1. When glycine was added to the enzymatic conversion reaction, the yield of the enzymatic conversion reaction was generally increased than when not added, and this effect was observed to be the highest when glycine was added at a concentration of 60 mM (Table 2) .

Table 2 below shows the yield of the enzymatic conversion reaction depending on the concentration of glycine during the enzymatic conversion reaction of the insulin glargine precursor.

**[Table 2]**

| | Concentration (mM) | Purity (%) | Yield (%) |
|---|---|---|---|
| Comparative Example 1 | - | 30.3 | 42.4 |
| Glycine | 20 | 31.6 | 43.1 |
| | 60 | 31.4 | 43.3 |
| | 100 | 30.8 | 42.8 |
| | 140 | 30.8 | 42.2 |

As shown in Table 2, the yield and purity were varied depending on the concentration of glycine. Specifically, the purity was increased from 30.3% before the addition of glycine to 31.6% when added at a concentration of 20 mM and to 31.4% when added at a concentration of 60 mM, but the extent of increase in purity was slightly decreased when added at concentrations of 100 mM and 140 mM. In addition, the yield was also increased from 42.4% before the addition of glycine to 43.1% when added at a concentration of 20 mM and to 43.3% when added at a concentration of 60 mM, but the extent of increase in yield was slightly decreased when added at concentrations of 100 mM and 140 mM. From these results, it was found that the yield and purity were effectively increased when glycine was added at an appropriate concentration during the enzymatic conversion reaction.

### Example 4. Improvement in yield of enzymatic conversion reaction by addition of proline

In order to confirm the effect of the addition of proline on the yield of the enzymatic conversion reaction, the insulin glargine precursor obtained in Example 1-3 in the concentration range of 1 to 15 mg/mL was added with trypsin corresponding to 1/100,000-1/1,000 of the weight ratio of the protein, followed by stirring for 2 hours. Here, the reaction solution was 50 mM Tris-HCl, proline was added thereto in the concentration range of 20 to 80 mM, and the pH was titrated to 8.5-9.0. After completion of stirring, the pH was titrated to 2.5 using acetic acid to complete the reaction.

The purity and yield of the completed insulin glargine were analyzed using RP-HPLC as in Comparative Example 1. When proline was added during the enzymatic conversion reaction, the yield of the enzymatic conversion reaction was generally increased than when not added, and this effect was observed to be the highest when proline was added at a concentration of 60 mM (Table 3) .

Table 3 below shows the yield of the enzymatic conversion reaction depending on the concentration of proline during the enzymatic conversion reaction of the insulin glargine precursor.

**[Table 3]**

| | Concentration (mM) | Purity (%) | Yield (%) |
|---|---|---|---|
| Comparative Example 1 | - | 35.8 | 41.6 |
| Proline | 20 | 37.8 | 43.1 |
| | 40 | 38.0 | 43.0 |
| | 60 | 37.6 | 43.2 |
| | 80 | 36.8 | 42.8 |

As shown in Table 3, when proline was added, the purity and yield of insulin glargine were increased. Accordingly, it was confirmed that the addition of proline had an influence on increasing the purity and yield in the enzymatic conversion reaction of insulin glargine.

Example 5. Improvement in yield of enzymatic conversion reaction by addition of calcium chloride/glycine/proline

In order to confirm the effect of the addition of calcium chloride, glycine, and proline on the yield of the enzymatic conversion reaction, the insulin glargine precursor obtained in Example 1-3 in the concentration range of 1 to 15 mg/mL was added with trypsin corresponding to 1/100,000-1/1,000 of the weight ratio of the protein, followed by stirring for 2 hours. Here, the reaction solution was 50 mM Tris-HCl, the concentration of calcium chloride/glycine/proline complex salt was set as shown in Table 4 below, and the pH was titrated to 8.5-9.0. After completion of stirring, the pH was titrated to 2.5 using acetic acid to complete the reaction.

**[Table 4]**

| Calcium chloride Concentration (mM) | Proline concentration (mM) | Glycine concentration (mM) |
|---|---|---|
| 30.0 | 10.0 | 50.0 |
| 20.0 | 20.0 | 35.0 |
| 20.0 | 20.0 | 50.0 |
| 20.0 | 20.0 | 50.0 |
| 20.0 | 20.0 | 50.0 |
| 10.0 | 10.0 | 50.0 |
| 20.0 | 3.2 | 35.0 |
| 20.0 | 36.8 | 35.0 |
| 20.0 | 20.0 | 60.2 |
| 10.0 | 30.0 | 50.0 |
| 30.0 | 30.0 | 50.0 |
| 0.0 | 0.0 | 0.0 |

The purity and yield of the completed insulin glargine were analyzed using RP-HPLC as in Comparative Example 1. When the calcium chloride/glycine/proline complex salt was added during the enzymatic conversion reaction, the yield of the enzymatic conversion reaction was generally increased than when not added, and this effect was observed to be the highest when 30 mM calcium chloride, 30 mM proline, and 50 mM glycine were added (Table 5).

Table 5 below shows the yield of the enzymatic conversion reaction depending on the concentration of calcium chloride/glycine/proline during the enzymatic conversion reaction of the insulin glargine precursor.

**[Table 5]**

| Calcium chloride Concentration (mM) | Proline concentration (mM) | Glycine concentration (mM) | Purity (%) | Yield (%) |
|---|---|---|---|---|
| 30.0 | 10.0 | 50.0 | 39.8 | 44.8 |
| 20.0 | 20.0 | 35.0 | 38.8 | 44.8 |
| 20.0 | 20.0 | 50.0 | 39.0 | 44.9 |
| 20.0 | 20.0 | 50.0 | 39.2 | 44.6 |
| 20.0 | 20.0 | 50.0 | 39.1 | 44.9 |
| 10.0 | 10.0 | 50.0 | 38.6 | 44.5 |
| 20.0 | 3.2 | 35.0 | 39.1 | 44.8 |
| 20.0 | 36.8 | 35.0 | 39.8 | 44.9 |
| 20.0 | 20.0 | 60.2 | 39.9 | 44.8 |
| 10.0 | 30.0 | 50.0 | 38.6 | 44.4 |
| 30.0 | 30.0 | 50.0 | 39.7 | 45.0 |
| 0.0 | 0.0 | 0.0 | 33.2 | 39.6 |

As shown in Table 5, the yield and purity were increased depending on the concentration of the calcium chloride/glycine/proline complex salt. It was confirmed that the purity was increased by 5.4-6.8% and the yield was increased by 4.8-5.4% when three salts were added together than when added separately. Therefore, it was found that the addition of calcium chloride/glycine/proline was very effective at increasing the purity and yield of the enzymatic conversion reaction of insulin glargine.

From the above description, those skilled in the art to which the present invention belongs will appreciate that the present invention may be embodied in other specific forms without changing the technical spirit or essential features thereof. In this regard, the embodiments described above should be understood to be non-limiting and illustrative in every way. The scope of the present invention is defined by the claims below rather than the aforementioned detailed description, and all changes or modified forms that are capable of being derived from the meaning, range, and equivalent concepts of the appended claims should be construed as being comprised in the scope of the present invention.

### [Industrial Applicability]

According to the present invention, a composition for enzymatic conversion of an insulin precursor into insulin and a method for converting an insulin precursor into insulin using the same make it possible to simultaneously improve both the structural stability of an insulin protein and the stability of an enzyme, thereby notably increasing the yield of enzymatic conversion and the purity of insulin, and thus can be usefully applied to the process of production of insulin.

### [Sequence List Free Text]

An electronic file is attached.

## Claims

1. A composition for enzymatic converting an insulin precursor into insulin, comprising one or more selected from the group consisting of calcium chloride, glycine, and proline; and trypsin and/or carboxypeptidase B.

2. The composition according to claim 1, further comprising a buffer solution.

3. The composition according to claim 2, wherein the buffer solution is 1-100 mM Tris-HCl or 1-100 mM borate.

4. The composition according to claim 1, wherein the trypsin is comprised at a weight ratio of 1/1,000 to 1/40,000 relative to the insulin precursor, and(or) the carboxypeptidase B is comprised at a weight ratio of 1/600 to 1/20,000 relative to the insulin precursor.

5. The composition according to claim 1, wherein the insulin is insulin glargine.

6. The composition according to claim 1, wherein the composition comprises calcium chloride, glycine, and proline.

7. The composition according to claim 1 or 6, wherein calcium chloride is comprised at 5-30 mM, glycine is comprised at 3-60 mM, and proline is comprised at 20-80 mM.

8. A method for converting an insulin precursor into insulin, comprising adding an insulin precursor to the composition according to any one of claims 1 to 6 and carrying out a reaction.

9. The method according to claim 8, wherein calcium chloride is comprised at 5-30 mM, glycine is comprised at 3-60 mM, and proline is comprised at 20-80 mM.


<110>     POLUS Inc.

<120>     An Enzymatic Conversion Composition for Producing Insulin from
          Proinsulin and a Method for Producing Insulin from Proinsulin
          Using the Same

<130>     PP-B2347

<150>     KR 10-2018-0170529
<151>     2018-12-27

<160>     4

<170>     KoPatentIn 3.0

<210>     1
<211>     21
<212>     PRT
<213>     Artificial Sequence

<220>
<223>     Insulin A chain


<400>     1
Gly Ile Val Glu Gln Cys Cys Thr Ser Ile Cys Ser Leu Tyr Gln Leu
  1               5                  10                  15

Glu Asn Tyr Cys Asn
            20


<210>     2
<211>     30
<212>     PRT
<213>     Artificial Sequence

<220>
<223>     Insulin B chain


<400>     2
Phe Val Asn Gln His Leu Cys Gly Ser His Leu Val Glu Ala Leu Tyr
  1               5                  10                  15

Leu Val Cys Gly Glu Arg Gly Phe Phe Tyr Thr Pro Lys Thr
            20                  25                  30


<210>     3
<211>     21
<212>     PRT
<213>     Artificial Sequence

<220>
<223>     Insulin glargine A chain


<400>     3
Gly Ile Val Glu Gln Cys Cys Thr Ser Ile Cys Ser Leu Tyr Gln Leu
  1               5                  10                  15

Glu Asn Tyr Cys Gly

            20

<210> 4
<211> 32
<212> PRT
<213> Artificial Sequence

<220>
<223> Insulin glargine B chain

<400> 4
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30